(19) Europäisches Patentamt
European Patent Office
Office européen des brevets

(11) Veröffentlichungsnummer : **0 003 517**
**B1**

(12) # EUROPÄISCHE PATENTSCHRIFT

(45) Veröffentlichungstag der Patentschrift : **25.03.81**

(51) Int. Cl.³ : **C 07 C 69/67**, C 07 C121/75, A 01 N 37/38

(21) Anmeldenummer : **79100172.0**

(22) Anmeldetag : **22.01.79**

(54) **Cyanomethylester der 4-(p-Trifluormethylphenoxy)-alpha-phenoxy-propionsäure, Verfahren zu seiner Herstellung und ihn als Wirkstoff enthaltendes herbizides Mittel und dessen Verwendung.**

(30) Priorität : **03.02.78 CH 1214/78**

(43) Veröffentlichungstag der Anmeldung : **22.08.79 (Patentblatt 79/17)**

(45) Bekanntmachung des Hinweises auf die Patenterteilung : **25.03.81 Patentblatt 81/12**

(84) Benannte Vertragsstaaten : **BE CH DE FR GB IT NL**

(56) Entgegenhaltungen :
**DE - A - 2 628 384**
**DE - A - 2 730 591**
**DE - A - 2 809 541**
**FR - A - 2 348 182**
**US - A - 4 059 435**

(73) Patentinhaber : **CIBA-GEIGY AG**
**Patentabteilung Postfach**
**CH-4002 Basel (CH)**

(72) Erfinder : **Böhner, Beat, Dr.**
**Hügelweg 3**
**CH-4102 Binningen (CH)**
Erfinder : **Rohr, Otto, Dr.**
**Kilbertweg 19**
**CH-4106 Therwil (CH)**

**EP 0 003 517 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Cyanomethylester der 4-(p-Trifluormethylphenoxy)-alpha-phenoxy-propionsäure, Verfahren zu seiner Herstellung und ihn als Wirkstoff enthaltendes herbizides Mittel und dessen Verwendung

Die vorliegende Erfindung betrifft einen neuen herbizid wirksamen Ester, nämlich den Cyanomethylester der 4-(p-Trifluormethyl-phenoxy)-α-phenoxy-propionsäure, Verfahren zur Herstellung dieses Esters, ferner ein herbizides Mittel, das diesen neuen Ester als Wirkstoff enthält, sowie die Verwendung des neuen Esters und ihn enthaltender Mittel zur selektiven Bekämpfung von insbesondere grasartigen Unkräutern in dikotylen Kulturpflanzenbeständen.

Aus den deutschen Offenlegungsschriften 1 912 600, 2 311 638, 2 613 675, 2 613 697 und 2 639 796 sowie aus den US-Patentschriften 3 322 525 und 4 059 435 sind schon halogenierte und/oder trifluormethylierte Cyanodiphenyläther und Cyanoalkoxydiphenyläther als herbizide Wirkstoffe bekannt geworden. Die Cyanoaethylester der 4-(p-Trifluormethylphenoxy)-α-phenoxy-propionsäure sind im belgischen Patent No. 856101 und in der DOS 2628384 beschrieben worden.

Die vorgenannten Wirkstoffe besitzen bei tiefen Aufwandmengen eine relativ schwache Wirkung gegen grasartige (monocotyle) Unkräuter bei pre- oder postemergenter Applikation oder sind in dicotylen Kulturpflanzenbeständen nicht selektiv, eignen sich also nur bedingt zur selektiven Bekämpfung von Ungräsern in dicotylen Kulturpflanzen.

Es wurde nun überraschenderweise gefunden, dass der Cyanomethylester der 4-(p-Trifluormethylphenoxy)-α-phenoxy-propionsäure eine viel bessere Wirkung gegen Gräser (monocotyle Pflanzen) besitzt und dicotyle Kulturen praktisch nicht schädigt, also zur Bekämpfung von Ungräsern in dicotylen Kulturpflanzenbeständen, wie Baumwolle, Soja, Zuckerrübe, Leguminosen etc. sehr gut geeignet ist ; besonders überraschend ist, dass er auch dem entsprechenden Cyanoäthylester der DOS 2 628 384 in der Bekämpfung von resistenten Ungräsern (Avena fatua) klar überlegen ist.

Der neue Ester vorliegender Erfindung besitzt die Formel I

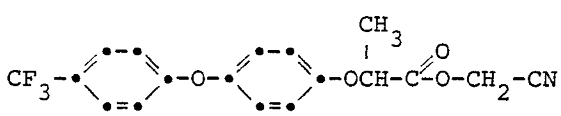 (I)

zur Herstellung des neuen Esters der Formel I dienen an sich bekannte Methoden.

Nach einem ersten Verfahren setzt man ein 4-(p-Trifluormethylphenoxy)-α-phenoxy-propionsäurehalogenid der Formel II

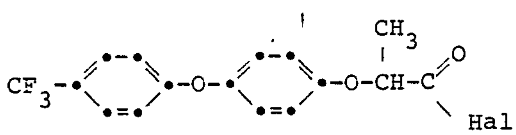 (II)

worin « Hal » Chlor oder Brom darstellt, in Gegenwart eines basischen Säureakzeptors mit Cyanomethanol (Glycolsäurenitril)

$$HO\text{-}CH_2\text{-}CN$$

um.

Gemäss einem weiteren Verfahren setzt man den Hydroxydiphenyläther oder ein Salz desselben, von der Formel III

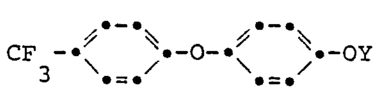 (III)

worin Y Wasserstoff oder das Kation eines Alkalimetalls bedeutet, mit einem α-Halogenpropionsäurecyanomethylester der Formel IV

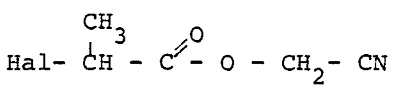 (IV)

worin « Hal » Chlor oder Brom darstellt, in Gegenwart eines säurebindenden Mittels um.

Die Ausgangsstoffe der Formeln II und III sind bekannt. IV kann gemäss Beispiel 2a) hergestellt werden.

Die Umsetzungen werden vorzugsweise in einem gegenüber den Reaktionskomponenten inerten

Lösungs- oder Verdünnungsmittel durchgeführt. Dazu eignen sich solche aus den verschiedensten Stoffklassen, wie aliphatische und aromatische, gegebenenfalls chlorierte Kohlenwasserstoffe, sowie polare inerte organische Lösungsmittel, wie Aether, Ketone, Amide, stabile Ester, wie z.B. Methylenchlorid, Methyl-äthyl-keton, Dimethoxyäthan, Dimethylformamid, Dimethylsulfoxid, Tetrahydrofuran etc.

Als basische Säureakzeptoren (Säurebindemittel) für die Umsetzungen mit den Halogenverbindungen der Formel II und IV können wässerige Alkalimetallhydroxide, wie KOH und NaOH sowie weitere übliche basische Stoffe, wie Karbonate ($K_2CO_3$, $NaHCO_3$) Alkoholate ($NaOCH_3$ und Kalium-tert. butylat), aber insbesondere auch organische Basen, wie Triäthylamin etc. verwendet werden.

Die nachfolgenden Beispiele veranschaulichen die Herstellung des erfindungsgemässen Cyanomethylesters der Formel I.

## Beispiel 1

17,2 g (0,05 Mol) 4-(4'-Trifluormethylphenoxy)-α-phenoxy-propionsäurechlorid werden bei 10 °C zu einem Gemisch von 3,13 g (0,055 Mol) 100 %-igem Glycolsäurenitril (Cyanomethanol) 5,5 g (0,054 Mol) Triäthylamin in 50 ml Methylenchlorid getropft. Anschliessend lässt man 1 Std. bei Raumtemperatur ausrühren, gibt 100 ml Wasser zu und trennt die organische Phase ab. Diese wird direkt über eine kleine Kieselgelsäule filtriert und eingedampft. Man erhält ein festes Produkt, welches man mit Petroläther verreibt und abfiltriert. Nach dem Trocknen erhält man 13,2 g (72,5 %) 4-(4'-Trifluormethylphenoxy)-α-phenoxy-propionsäure-cyanomethylester mit dem Fp. 53-56 °C.

## Beispiel 2

a) 215,9 g (1,0 Mol) α-Brompropionsäurebromid werden in 500 ml Dimethoxyäthan gelöst und bei 10 °C langsam mit 62,8 g (1,1 Mol) Glycolsäurenitril versetzt. Anschliessend tropft man bei 10-20 °C 111,3 (1,1 Mol) Triäthylamin zu. Das Reaktionsgemisch wird 10 Min. bei Raumtemperatur ausgerührt und über Hyflo ®abgenutscht. Das Filtrat dampft man ein und destilliert den Rückstand am Wasserstrahlvacuum. Als Hauptfraktion erhält man 107,0 g (55,7 %) α-Brompropionsäure-cyanomethylester mit dem Sdp. 109 °C/17 Millibar. und der Formel

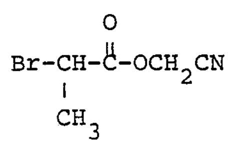

b) 15,2 g (0,06 Mol) 4-(4'-Trifluormethylphenoxy)-phenol werden mit 9,7 g (0,07 Mol) Kaliumcarbonat in 50 ml Methyläthylketon für 2 Std. am Rückfluss gerührt. Danach gibt man bei 50 °C 12,1 g (0,063 Mol) des gemäss a) hergestellten α-Brompropionsäure-cyanomethylesters zu und lässt 15 Std. bei 50 °C ausrühren. Das Reaktionsgemisch wird abfiltriert und eingedampft. Den Rückstand löst man in Methylenchlorid und filtriert die Lösung über eine kleine Kieselgelsäule ab. Dann wird sie eingedampft und man erhält nach dem Trocknen 16,6 g (76 %) 4-(4'-Trifluormethylphenoxy)-α-phenoxy-propionsäure-cyanomethylester, welcher mit dem gemäss Beispiel 1 hergestellten identisch ist und bei 53-56 °C schmilzt.

Der erfindungsgemässe Wirkstoff ist eine stabile Verbindung, welche in üblichen organischen Lösungsmitteln, wie Alkanolen, Ketonen, Aethern, Dimethylformamid, Dimethylsulfoxyd etc. löslich ist.

Die Erfindung betrifft auch herbizide Mittel, welche den neuen Wirkstoff der Formel I enthalten, sowie Verfahren zur pre- und post-ermergenten Unkrautbekämpfung, insbesondere von monocotylen Ungräsern.

Die erfindungsgemässen Mittel können in den üblichen Formulierungen vorliegen.

Die Herstellung erfindungsgemässer Mittel erfolgt in an sich bekannter Weise durch inniges Vermischen und Vermahlen des Wirkstoffs der Formel I mit geeigneten Trägerstoffen und/oder Verteilungsmitteln, gegebenenfalls unter Zusatz von gegenüber dem Wirkstoff inerten Antischaum-, Netz-, Dispersions- oder Lösungsmitteln. Der Wirkstoff kann in den folgenden Aufarbeitungsformen vorliegen und angewendet werden :

feste Aufarbeitungsformen : Stäubemittel, Streumittel, Granulate, Umhüllungsgranulate, Imprägnierungsgranulate und Homogengranulate :

in Wasser dispergierbare Wirkstoffkonzentrate : Spritzpulver, (wettable powder), Pasten, Emulsionen ; Emulsionskonzentrate ;

flüssige Aufarbeitungsformen : Lösungen.

Die Wirkstoffkonzentration beträgt in den erfindungsgemässen Mitteln 1 bis 80 Gew.-% und kann vor

der Anwendung auch auf niedrige Konzentrationen, wie etwa 0,05 bis 1 % verdünnt werden. Die Aufwandmengen betragen in der Regel 0,25 kg Wirkstoff pro Hektar, vorzugsweise 0,25 bis 4 kg/ha.

Die Herstellung erfindungsgemässer Mittel wird durch die folgenden Formulierungsbeispiele veranschaulicht. Teile bedeuten darin Gewichtsteile.

Granulat

Zur Herstellung eines 5 % igen Granulates werden die folgenden Stoffe verwendet :

5 Teile des erfindungsgemässen Esters der Formel I,
0,25 Teile Epichlorhydrin,
0,25 Teile Cetylpoläthylenglykoläther mit 8 Mol Aethylenoxid,
3,50 Teile Polyäthylenglykol,
91 Teile Kaolin (Korngrösse 0,3 bis 0,8 mm).

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

Spritzpulver

Zur Herstellung eines a) 50 % igen, b) 25 % igen und c) 10 % igen Spritzpulvers werden folgende Bestandteile verwendet :

a) 50 Teile des erfindungsgemässen Wirkstoffs,
5 Teile Natriumdibutylnaphthylsulfonat,
3 Teile Naphthalinsulfonsäuren-Phenonolsulfonsäuren-Formaldehyd-Kondensat 3 : 2 : 1,
20 Teile Kaolin,
22 Teile Champagne-Kreide ;

b) 25 Teile des obigen Wirkstoffes,
5 Teile Oleylmethyltaurid-Natrium-Salz,
2,5 Teile Naphthalinsulfonsäuren-Formaldehyd-Kondensat,
0,5 Teile Carboxymethylcellulose,
5 Teile neutrales Kalium-Aluminium-Silikat,
62 Teile Kaolin ;

c) 10 Teile des obigen Wirkstoffs,
3 Teile Gemisch der Natriumsalze von gesättigten Fettalkoholen,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Ungräsern in Kulturpflanzungen verwendet.

Paste

Zur Herstellung einer 45 % igen Paste werden folgende Stoffe verwendet :

45 Teile Wirkstoff,
5 Teile Natriumaluminiumsilikat,
14 Teile Cetylpolyäthylenglykoläther mit 8 Mol Aethylenoxid,
1 Teile Oleylpolyäthylenglykoläther mit 5 Mol Aethylenoxid,
2 Teile Spindelöl,
23 Teile Wasser,
10 Teile Polyäthylenglykol.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25 % igen Emulsionskonzentrates werden

25 Teile Wirkstoff,
10 Teile Mischung von Nonylphenolpolyoxyäthylen und Calcium-dodecylbenzol-sulfonat,

10 Teile Cyclohexanon,
55 Teile Xylol.
miteinander vermischt. Dieses Konzentrat kann mit Wasser zu Emulsionen auf geeignete Konzentrationen verdünnt werden.

Erfindungsgemässe Mittel, die als aktive Komponente den neuen Ester der Formel I enthalten, eignen sich besonders zur selektiven Bekämpfung monocotyler Ungräser in pre- und insbesondere postemergenter Anwendung in dicotylen Kulturpflanzenbeständen, wie z.B. Soja, Baumwolle, Zuckerrübe, Leguminosen, Klee, Luzerne, Melone, Gurke, Tabak usw.

Zum Nachweise der Brauchbarkeit als Herbizid (pre- und postemergent) dienen folgende Testmethoden :

Pre-emergente Herbizid-Wirkung (Keimhemmung)

Im Gewächshaus wird unmittelbar nach der Einsaat der Versuchspflanzen in Saatschalen die Erdoberfläche mit einer wässerigen Dispersion des Wirkstoffs, erhalten aus einem 25 %-igen Spritzpulver oder Emulsionskonzentrat behandelt. Es wurden vier verschiedene Konzentrationsreihen angewendet, entsprechend 4, 2, 1 und 0,5 kg Wirksubstanz pro Hektar. Die Saatschalen werden im Gewächshaus bei 22-25 °C und 50-70 % rel. Luftfeuchtigkeit gehalten und der Versuch nach 3 Wochen ausgewertet und die Resultate nach folgender Notenskala bonitiert :

1 = Pflanze nicht gekeimt oder total abgestorben
2-3 = sehr starke Wirkung
4-6 = mittlere Wirkung
7-8 = geringe Wirkung
9 = keine Wirkung (wie unbehandelte Kontroll)

als Versuchspflanzen dienen :

beta vulgaris (Zuckerrübe)
glycine (Soja)
gossypium (Baumwolle)
avena fatua
lolium perenne
alopecurus myosuroides
bromos tectorum
cyperus esculentus
rottboellia exaltata
digitaria sanguinalis
setaria italica
echinochloa crus galli

Post-emergente Herbizid-Wirkung (Kontaktherbizid)

Eine grössere Anzahl monocotyle Unkräuter und dicotyle Kulturpflanzen wurden nach dem Auflaufen (im 4- bis 6-Blattstadium) mit einer wässrigen Wirkstoffdispersion in Dosierungen von 0,125 ; 0,25 ; 0,5 ; 1 und 2 kg Wirksubstanz pro Hektar auf die Pflanzen gespritzt und diese bei 24°-26 °C und 45-60 % rel. Luftfeuchtigkeit gehalten. 15 Tage nach Behandlung wird der Versuch ausgewertet und das Ergebnis wie im pre-emergent-Versuch nach derselben Notenskala bonitiert.

Resultate des post-emergent-Versuches

| Pflanze | Aufwandmenge an Wirkstoff der Formel I kg/ha | | | | |
|---|---|---|---|---|---|
| | 2 | 1 | 0,5 | 0,25 | 0,125 |
| Soja (Kulturpflanze) | 9 | 9 | 9 | 9 | 9 |
| Lolium | 1 | 1 | 2 | 2 | 3 |
| Alopecurus | 1 | 1 | 1 | 2 | 2 |
| Bromus | 1 | 1 | 1 | 2 | 2 |
| Rottboellia | 1 | 1 | 1 | 1 | 1 |
| Digitaria | 1 | 1 | 1 | 1 | 1 |
| Setaria | 1 | 1 | 1 | 1 | 1 |
| Echinochloa | 1 | 1 | 1 | 1 | 1 |

In einem Vergleichsversuch wurde die selektive herbizide Wirkung des erfindungsgemässen Cyanomethylesters der Formel I in Zuckerrüben mit Avena fatua als Unkraut in einem weiteren postemergent-Test ermittelt und mit folgenden, Cyanogruppen enthaltenden strukturell nächstverwandten Wirkstoffen des Standes der Technik verglichen

Verbindung A : 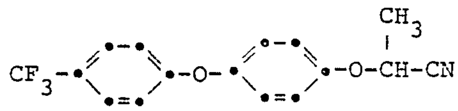 (DOS 2639796 Bsp. 39)

Verbindung B : 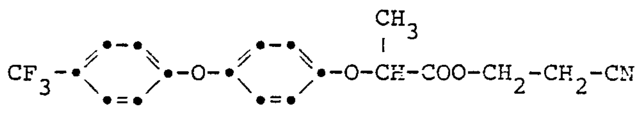 (DOS 2628384 Bsp. 66)

Die Aufwandmengen in diesem Versuch betrugen jeweils 0,125 kg/ha. Die Versuchsmethodik und Bonitierung waren identisch mit vorhergehendem Versuch.

Resultate

| Verbindung | Avena fatua | Zuckerrübe |
|---|---|---|
| Erfindungsgemässer Cyanomethylester (Formel I) | 3 | 9 |
| A | 9 | 9 |
| B | 8 | 9 |

Obiger Versuch zeigt deutlich, dass die strukturell nächstverwandten bekannten Verbindungen A und B die monocotyle Unkrautpflanze Avena fatua (Wildhafer) bei der verwendeten Aufwandmenge von 0,125 kg/ha praktisch überhaupt noch nicht schädigen, während der erfindungsgemässe Cyanomethylester schon sehr starke Schädigung der Unkrautpflanze bewirkt, in der selektiven Wirkung den beiden Vergleichssubstanzen also klar überlegen ist.

**Ansprüche**

1. Die herbizid wirksame Verbindung 4-(p-Trifluor-methylphenoxy)-α-phenoxy-propionsäure-cyanomethylester der Formel I

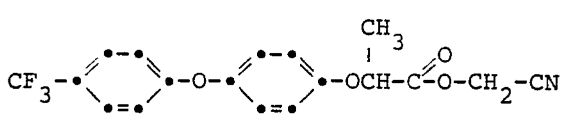 (I)

2. Verfahren zur Herstellung des neuen Esters der Formel I des Patentanspruchs 1, dadurch gekennzeichnet, dass man ein 4-(p-Trifluormethyl-phenoxy)-α-phenoxy-propionsäurehalogenid der Formel II

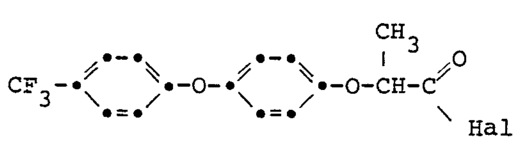 (II)

worin « Hal » Chlor oder Brom darstellt, in Gegenwart eines basischen Säureakzeptors mit Cyanomethanol (Glykolsäurenitril) der Formel $HO-CH_2-CN$ umsetzt.

3. Verfahren zur Herstellung des neuen Esters der Formel I des Patentanspruchs 1, dadurch gekennzeichnet, dass man den Hydroxy-diphenyläther oder ein Salz desselben, von der Formel III

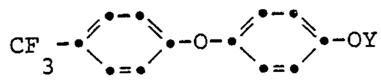 (III)

worin Y Wasserstoff oder das Kation eines Alkalimetalls bedeutet, in Gegenwart eines säurebindenden Mittels mit einem α-Halogenpropionsäure-cyanomethylester der Formel IV

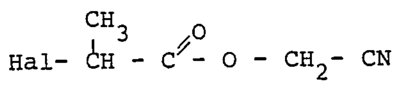 (IV)

umsetzt, worin « Hal » Chlor oder Brom darstellt.

4. Herbizides Mittel, dadurch gekennzeichnet, dass es als wirksame Komponente den 4-(p-Trifluormethylphenoxy)-α-phenoxy-propionsäure-cyanomethylester der Formel I des Patentanspruchs 1 enthält.

5. Die Verwendung des neuen Cyanomethylesters der Formel I zur Bekämpfung von monocotylen Unkräutern.

6. Die Verwendung des Cyanomethylesters der Formel I zur selektiven Bekämpfung von Ungräsern in dicotylen Kulturpflanzenbeständen, in vorzugsweise post-emergenter Applikation.

7. Verfahren zur selektiven Bekämpfung von monocotylen Unkräutern in dicotylen Kulturpflanzenbeständen, dadurch gekennzeichnet, dass man die verunkrauteten Kulturpflanzen vorzugsweise post-emergent mit einer wirksamen Menge eines Mittels gemäss Anspruch 4 behandelt, welches als aktive Komponente den Cyanomethylester der 4-(p-Trifluormethylphenoxy)-α-phenoxy-propionsäure enthält.

**Claims**

1. The herbicidally active 4-(p-trifluoromethylphenoxy)-α-phenoxypropionic acid cyanomethyl ester of the formula I

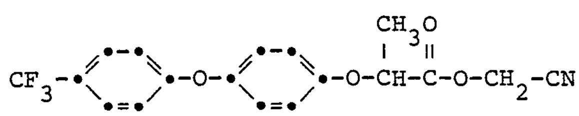 (I)

2. A process for the manufacture of the ester of the formula I of claim 1, which process comprises reacting a 4-(p-trifluoromethylphenoxy)-α-phenoxypropionic acid halide of the formula II

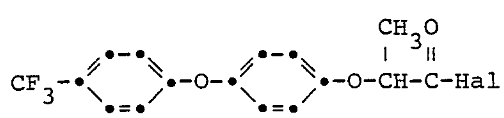 (II)

wherein Hal represents chlorine or bromine, in the presence of a basic acid acceptor, with cyanomethanol (hydroxyacetonitrile) of the formula $HO-CH_2-CN$.

3. A process for the manufacture of the ester of the formula I of claim 1, which process comprises reacting the hydroxydiphenyl ether, or a salt thereof, of the formula III

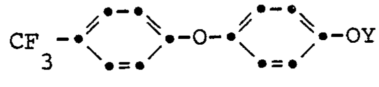 (III)

wherein Y represents hydrogen or the cation of an alkali metal, in the presence of an acid acceptor, with an α-halopropionic acid cyanomethyl ester of the formula IV

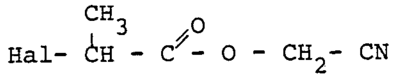 (IV)

wherein Hal represents chlorine or bromine.

4. A herbicidal composition which contains, as active component, the 4-(p-trifluoromethylphenoxy)-α-phenoxypropionic acid cyanomethyl ester of the formula I of claim 1.

5. A method of controlling monocotyledonous weeds at a locus, which comprises applying to said locus the cyanomethyl ester of the formula I.

6. A method of selectively controlling gramineous weeds in crops of dicotyledonous plants, which comprises applying thereto, preferably postemergence, the cyanomethyl ester of the formula I.

7. A method of selectively controlling monocotyledonous weeds in crops of dicotyledonous cultivated plants, which comprises preferably treating the weed-infested crops of cultivated plants postemergence with an effective amount of a composition according to claim 4, which contains, as active component, the cyanomethyl ester of 4-(p-trifluoromethylphenoxy)-α-phenoxypropionic acid.

**Revendications**

1. Le composé, possédant une activité herbicide, ester cyanométhylique de l'acide 4-p-trifluorométhylphénoxy)-alpha-phénoxy-propionique, de formule I

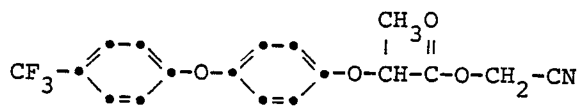 (I)

2. Procédé de préparation du nouvel ester de formule I de la revendication 1, caractérisé en ce que l'on fait réagir un halogénure de l'acide 4-(p-trifluorométhylphénoxy)-alpha-phénoxy-propionique de formule II

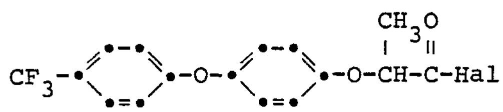 (II)

dans laquelle « Hal » représente le chlore ou le brome, en présence d'un accepteur d'acides basiques, avec le cyanométhanol (nitrile glycolique) de formule $HO-CH_2-CN$.

3. Procédé de préparation du nouvel ester de formule I de la revendication 1, caractérisé en ce que l'on fait réagir l'éther hydroxydiphénylique, ou un sel de cet éther, de formule III

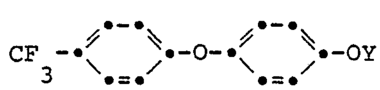 (III)

dans laquelle Y représente l'hydrogène ou le cation d'un métal alcalin, en présence d'un agent fixant les acides, avec un alpha-halogénopropionate de cyanométhyle de formule IV

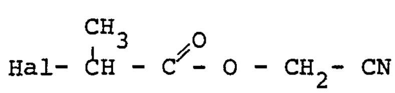 (IV)

dans laquelle « Hal » représente le chlore ou le brome.

4. Produit herbicide caractérisé en ce qu'il contient en tant que composant actif le 4-(p-trifluorométhylphénoxy)-alpha-phénoxy-propionate de cyanométhyle de formule I de la revendication 1.

5. L'utilisation du nouvel ester cyanométhylique de formule I pour la lutte contre les mauvaises herbes monocotylédones.

6. L'utilisation de l'ester cyanométhylique de formule I pour la lutte sélective contre les graminées parasitant les cultures de végétaux dicotylédones, de préférence en application en post-levée.

7. Procédé pour combattre sélectivement les mauvaises herbes monocotylédones dans les cultures de végétaux dicotylédones, caractérisé en ce que l'on traite les cultures envahies par les mauvaises herbes, de préférence en post-levée, par une quantité efficace d'un produit selon la revendication 4 contenant en tant que composant actif l'ester cyanométhylique de l'acide 4-(p-trifluorométhylphénoxy)-alpha-phénoxypropionique.